(19) Europäisches Patentamt
European Patent Office
Office européen des brevets

(11) **EP 1 810 708 B1**

(12) **EUROPEAN PATENT SPECIFICATION**

(45) Date of publication and mention
of the grant of the patent:
**18.11.2009 Bulletin 2009/47**

(51) Int Cl.:
***A61M 16/00*** (2006.01)

(21) Application number: **07000322.3**

(22) Date of filing: **15.09.2000**

(54) **Patient-ventilator synchronization using dual phase sensors**

Synchronisierung einer Beatmungsvorrichtung mittels Doppelphasensensoren

Synchronisation d'un appareil d'assistance respiratoire à détecteur double phase

(84) Designated Contracting States:
**DE FR GB SE**

(30) Priority: **15.09.1999 US 154196 P**

(43) Date of publication of application:
**25.07.2007 Bulletin 2007/30**

(62) Document number(s) of the earlier application(s) in accordance with Art. 76 EPC:
**00963813.1 / 1 229 956**

(73) Proprietor: **ResMed Ltd.**
**North Ryde, NSW 2113 (AU)**

(72) Inventor: **Berthon-Jones, Michael**
**Leonay, NSW 2750 (AU)**

(74) Representative: **Cockerton, Bruce Roger et al**
**Carpmaels & Ransford**
**43-45 Bloomsbury Square**
**London**
**WC1A 2RA (GB)**

(56) References cited:
**EP-A- 0 452 001      EP-A- 1 083 953**
**US-A- 5 803 065**

- **WARD M E; EIDELMAN D; STUBBING D G; BELLEMARE F; MACKLEM P T: "Respiratory sensation and pattern of respiratory muscle activation during diaphragm fatigue" JOURNAL OF APPLIED PHYSIOLOGY, vol. 65, no. 5, 1988, pages 2181-2189, XP009038387 UNITED STATES**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the Invention**

This invention relates to a device for providing ventilatory assistance to a patient. More specifically, the invention involves an improved device that provides ventilation in phase with a paetient's respiratory efforts through the use of a respiratory effort sensor.

**Background of the Invention**

Devices for providing mechanical ventilation to assist patient respiration are well known. Such ventilator devices have been used to help patients with such ailments as severe lung disease, chest wall disease, neuromuscular disease and other diseases of respiratory control. Generally, a ventilator provides air or oxygen-enriched air to a patient at pressures that are higher during inspiration and lower during expiration.

Several types of ventilator devices exist. These types include bi-level ventilators, proportional assist ventilators and servo-controlled ventilators. Each type of ventilator utilizes different methods for assisting with patient respiration and achieves different goals.

Bi-level ventilators provide the simplest level of support. These ventilators supply a mask pressure $P(t)$ which is higher by an amplitude $A$ from an initial pressure $P_0$ when respiratory airflow $f(t)$ is inspiratory, $f(t) > 0$, than when respiratory airflow is expiratory, $f(t) \leq 0$.

$$P(t) = P_0 + A \qquad f(t) > 0 \qquad \text{(inspiration)}$$

$$P(t) = P_0 \qquad\qquad \text{otherwise} \qquad \text{(expiration)}$$

Thus, these devices supply a fixed degree of support $A$. However, they do not guarantee any particular ventilation when, for example, the patient's efforts are inadequate.

Proportional assist ventilators represent an attempt to provide support more closely in phase with the patient's respiratory efforts. Proportional assist ventilators provide mask pressure as follows:

$$P(t) = P_0 + R\, f(t) + E_{LC} \int f(t)dt \qquad f(t) > 0 \qquad \text{(inspiration)}$$

$$P(t) = P_0 + R\, f(t) \qquad\qquad \text{otherwise} \qquad \text{(expiration)}$$

where $R$ is a substantial fraction of the patient's airway resistance, and $E_{LC}$ is a substantial fraction of the patient's lung plus chest wall elastance. So long as there is no leak, this provides support much more closely in phase with a patient's respiratory efforts. However, again, in the case of a patient's efforts being inadequate, for example, due to reduced chemoreflex control of breathing during sleep, there is no guaranteed minimum ventilation,

One method of ensuring an adequate degree of ventilatory support is to use a servo ventilator, which adjusts the degree of support $A$ to servo-control instantaneous ventilation $V(t)$ to equal a target ventilation $V_{TGT}$:

$P(t) = P_0 + A \qquad f(t) > 0$, or time since the start of the last inhalation $> T_{MAX}$

$P(t) = 0 \qquad$ otherwise

where:

$$A = G \int ( V(t) - V_{TGT} )\, dt$$

$$V(t) = 0.5 \, abs \, ( \, f(t) \, )$$

$$A_{MIN} < A < A_{MAX}.$$

In this ventilator, $V(t)$ is one half the absolute value of the respiratory airflow, G is the gain of the integral servo-controlter, a value of 0.3 cmH$_2$O per L/min error in ventilation per second being suitable, and $A_{MIN}$ and $A_{MAX}$ are limits set on the degree of support A for comfort and safety, 0.0 and 20.0 cmH$_2$O being generally suitable. Unfortunately, while this method achieves a guaranteed minimum ventilation, there is little attempt to keep the support precisely in phase with the patient's own respiratory efforts. Rather, the system merely makes a step change in pressure at the start and end of inspiration.

In a more advanced servo-controlled ventilator, both guaranteed ventilation and phase synchronization is achieved. This apparatus is the subject of the commonly owned International Patent Application entitled "Assisted Ventilation to Match Patient Respiratory Need," International Publication Number WO 98/12965 (hereinafter referred to as "AutoVPAP"). The AutoVPAP apparatus provides an instantaneous mask pressure $P(t)$ based upon a substantial fraction of the patient's airway resistance R, respiratory airflow $f(t)$, an amplitude A, and an estimation of the patient's instantaneous respiratory phase $\Phi$ as applied to a smooth pressure waveform template $\Pi(\Phi)$ as follows:

$$P(t) = P_0 + R \, f(t) + A \, \Pi(\Phi) \qquad \text{for all } f(t) \qquad \text{(inspiration and expiration)}$$

where:

$$A = G \int (V(t) - V_{TGT}) \, dt$$

$$V(t) = 0.5 \, abs \, ( \, f(t) \, )$$

$$A_{MIN} < A < A_{MAX}.$$

In estimating the respiratory phase, the AutoVPAP apparatus uses a respiratory airflow signal and its derivative as input data for a set of fuzzy logic rules that are associated with particular phases of respiration. Using the results of the evaluations of the rules, a single phase value is derived and used as the instantaneous respiratory phase. Thus, the degree of ventilatory support is varied in phase with the patient's respiration. Moreover, as the calculation of A is based upon a target ventilation $V_{TGT}$, a guaranteed level of ventilation is provided.

However, in this AutoVPAP system, room for improvement exists in the phase determination due to the problem of leak. Mask and/or mouth leak is ubiquitous during noninvasive ventilatory support using a mask, and is particularly problematical during sleep. Leak causes mis-measurement of the respiratory airflow, and therefore can severely interfere with patient-machine synchronization.

EP 0452001A discloses a method and associated apparatus for providing synchronized ventilatory support to a patient. The apparatus comprises:

a sensor to generate a respiratory airflow signal;
a processor for analyzing said respiratory airflow signal for determining the instantaneous respiratory phase of the patient and for generating a desired pressure request signal as a function of said instantaneous respiratory phase and a desired ventilation; and
a servo-controlled blower to provide pressurized air to said patient in accordance with said pressure request signal.

US 5,803,065 discloses a method and associated apparatus for adjusting pressure based on flow, but not on patient effort. The method relies upon a determination as to whether the patient is inhaling or exhaling, which is achieved by comparing instantaneous flow to an average.

EP 1083953, which comprises part of the state of the art under Article 54(3) EPC, discloses another method and associated apparatus for adjusting pressure based on flow, but not on patient effort. No measurement, calculation or other determination is made of patient effort, nor is patient effort used in any way to adjust ventilation.

Brief Description of the Invention

An objective of the present invention is to provide apparatus to supply ventilatory assistance precisely in phase with a patient's spontaneous ventilatory efforts provided these efforts are adequate to maintain a specified target ventilation.

A further objective of the present invention is to provide apparatus to guarantee at least a specified target ventilation, even in the event that the patient's efforts become irregular or cease.

Additional objectives will be apparent from the description of the invention as contained herein.

According to the invention, these is provided an apparatus as set forth in the accompanying claim 1.

In its broadest aspects, the present invention involves an improved ventilator which delivers ventilatory support that is synchronized with the phase of a patient's respiratory efforts and guarantees a targeted minimum ventilation. The device provides ventilatory support to a patient based in part upon a calculated instantaneous mask pressure using a general method similar to that of the previously described AutoVPAP system. However, the respiratory phase determination is improved by using data representing both respiratory airflow and respiratory effort.

More specifically, in estimating the respiratory phase, the device utilizes a respiratory airflow signal and preferably its rate of change. The degree of membership of the respiratory airflow signal in each of the fuzzy sets zero, positive, large positive, negative, and large negative is calculated using suitable membership functions. Similarly, the degree of membership of the derivative of the respiratory airflow signal in each of the fuzzy sets steady, increasing, increasing fast, decreasing and decreasing fast is calculated using suitable membership functions. The degrees of membership in these classes are used in a set of fuzzy logic inference rules. Each fuzzy inference rule is associated with a particular phase of respiration.

Although many variations are possible, in the preferred embodiment, the inference rules relating to respiratory airflow are as follows:

1. If the airflow is zero and increasing fast, then the phase is 0 revolutions.

2. If the airflow is large positive and steady, then the phase is 0.25 revolutions.

3. If the airflow is zero and falling fast, then the phase is 0.5 revolutions.

4. If the airflow is large negative and steady, then the phase is 0.75 revolutions.

5. If the airflow is zero and steady and the 5-second low-pass filtered absolute value of the respiratory airflow is large, then the phase is 0.9 revolutions.

6. If the airflow is positive and the phase is expiratory, then the phase is 0.1 revolutions.

7. If the airflow is negative and the phase is inspiratory, then the phase is 0.6 revolutions.

8. If the 5-second low-pass filtered absolute value of the respiratory airflow is small, then the phase in the respiratory cycle is increasing at a fixed rate equal to the patient's expected respiratory rate.

9. If the 5-second low-pass filtered absolute value of the respiratory airflow is large, then the phase in the respiratory cycle is increasing at a steady rate equal to the existing rate of change of phase, low-pass filtered with a time constant of 20 seconds.

In the invention, the device combines additional fuzzy inference rules based upon an effort signal from an effort sensor. The effort sensor is not dependent on measured airflow and as such is immune to errors associated with leak. The effort signal and preferably its rate of change are used as input values for membership functions for the additional fuzzy inference rules that are also associated with particular phases. The membership functions are used to calculate the degree of membership of the effort signal in each of the fuzzy sets zero, medium and large, and the degree of membership of its derivative in each of the fuzzy sets increasing moderately, increasing fast, decreasing moderately and decreasing fast.

In the preferred embodiment, the inference rules relating to respiratory effort are as follows:

10. If the effort signal is zero and increasing fast, then the phase is 0 revolutions.

11. If the effort signal is medium and increasing moderately, then the phase is 0.2 revolutions.

12. If the effort signal is large and decreasing fast, then the phase is 0.5 revolutions.

13. If the effort signal is medium and decreasing moderately, then the phase is 0.7 revolutions.
In general, the phase values need not be these exact values, but can approximate them.

In a process similar to that of the AutoVPAP system, all of the fuzzy inference rules are evaluated and a single phase value is derived and taken as the instantaneous respiratory phase of the patient. This allows the degree of support provided by the device to remain in phase with the patient's respiration.
In the preferred embodiment, the effort sensor is a suprasternal notch sensor. However, alternative embodiments of the invention may utilize other effort sensors including, for example, an esophageal pressure sensor or an electromyograph.

Brief Description of the Drawings

Fig. 1 depicts the components of a servo-controlled ventilator with an effort sensor, and

Fig. 2 depicts the steps involved in determining the delivered pressure level, including use of the effort signal.

Detailed Description of the invention

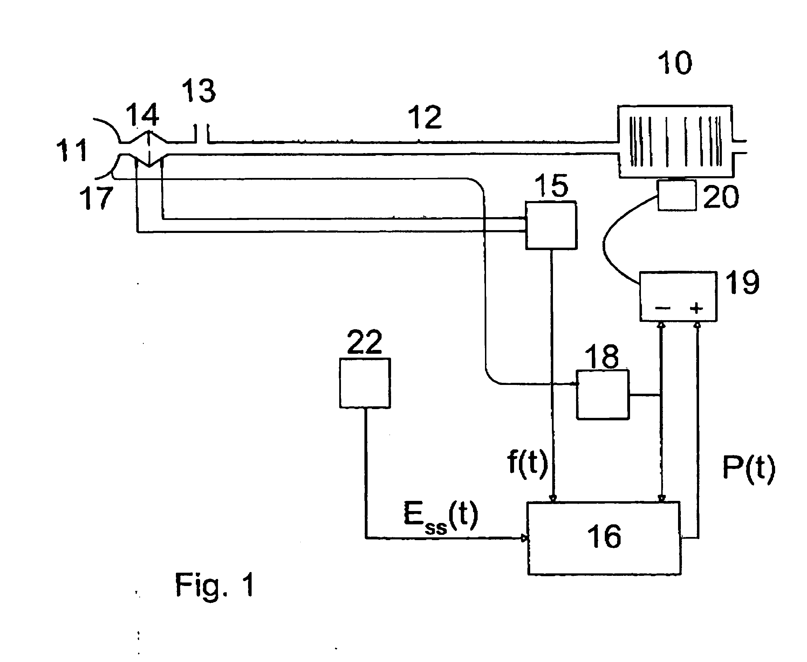

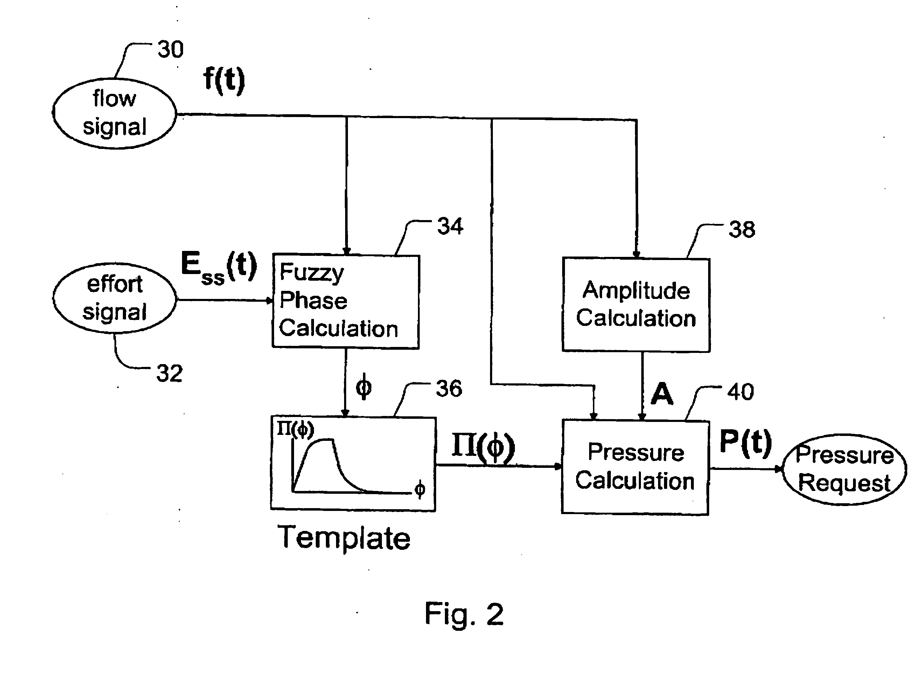

A servo-controlled ventilator useful for accomplishing the present invention is shown in Fig. 1. A blower 10 supplies air under pressure via delivery tube 12 to a mask 11. Exhaust gas is vented via exhaust 13. Mask flow is measured using pneumotachograph 14 and differential pressure transducer 15 to derive flow signal f(t). Mask pressure is measured at pressure tap 17 using pressure transducer 18. Respiratory effort is measured by an effort sensor 22 to yield an effort signal $E_{98}(t)$. Flow, effort and pressure signals are sent to microcontroller 16 which implements the processing shown in Fig. 2 to derive a pressure request signal P(t). The actual measured pressure and pressure request signal P(t) are fed to motor servo 19 which controls blower motor 20 to produce the desired instantaneous mask pressure. An example of this type of ventilator, without an effort sensor 22, is the subject of International Publication No. WO 98/12965. which is also disclosed in related U.S. Patent Serial No. 6,532,959. An additional example is disclosed in International Publication No. WO 99/61088, which is also contained in related U.S. Patent No. 6,532,959. The foregoing U.S. applications are hereby incorporated by reference.
In the preferred embodiment of the present invention, a suprasternal notch sensor is used as the effort sensor 22 to generate the effort signal. The sensor is more fully described in a commonly owned patent publication entitled "Measurement of Respiratory Effort Using a Suprasternal Sensor,' No US 6,758,216. The suprasternal notch sensor measures changes in the suprasternal notch. Increasing inspiratory efforts cause the skin of the suprasternal notch to retract. An optical sensor generates an electrical signal that is an increasing function of inspiratory effort derived by measuring changes in the depth of the skin of the suprasternal notch. In a further commonly owned patent publication, entitled "Ventilatory Assistance Using an External Effort Sensor," No US 6,445,942, the effort signal is used to trigger a bilevel ventilator.
With reference to Fig. 2, using the aforementioned system of Fig. 1, several steps are used to derive the pressure request signal P(t). In steps 30 and 32, an instantaneous airflow signal f(t) and an effort signal $E_{33}(t)$ are generated. In step 34, labeled Fuzzy Phase Calculation, the system calculates the phase Φ in the respiratory cycle as a continuous variable using a set of fuzzy inference rules. The fuzzy inference rules are based upon both respiratory airflow and respiratory effort.
The preferred rules with respect to respiratory airflow are those described above, rules 1-9, although many other inference rules can be developed based upon respiratory airflow. The fuzzy extents, or the degrees of truth, to which the airflow is in fuzzy sets zero, positive, large positive, negative, and large negative, and the 5-second low-pass filtered absolute value is small and large, are determined with suitable membership functions using the measured respiratory airflow f(t). Similarly, the fuzzy extents to which the airflow is in fuzzy sets steady, increasing fast and decreasing fast are determined with suitable membership functions using the change in respiratory airflow df(t)/dt. The fuzzy extents to which the phase is a member of the fuzzy sets expiratory and inspiratory is determined by membership functions using a previously calculated instantaneous phase. This approach using respiratory airflow to determine phase is that disclosed in International Publication Nos. WO 98/12965 and WO 99/61088.
Rules 1-4 estimate the phase directly from the instantaneous respiratory airflow. Rule 5 permits an expiratory pause, whose length may be long if the patient has recently been breathing adequately, and short or zero if the patient is not breathing. Rules 6-7 provide for quick resynchronization in the event that the patient breathes irregularly. Rule 8 provides the equivalent of a timed backup, in which, to the extent that the patient has stopped breathing or is not adequately breathing, the ventilator will cycle at a suitable fixed rate. Rule 9 provides that to the extent that the patient is breathing adequately, the ventilator will tend to track the patient's recent average respiratory rate. This is particularly appropriate for patients with cardiac failure and Cheyne-Stokes breathing, whose respiratory rates tend to be extremely steady despite rhythmic changes in amplitude.

An effect of the changing degree of activation of rules 8 and 9 is that, to the fuzzy extent that the instantaneous ventilation equals or exceeds the target ventilation, ventilatory support will be provided in phase with the patient's own respiratory efforts, and to the extent that the instantaneous ventilation is less than the target ventilation, ventilatory support will be provided at a pre-set rate.

In an elaboration of this embodiment, the weighting of rules 1-6 can be made proportional to the fuzzy extent that the instantaneous ventilation is large compared with the target ventilation, thereby reinforcing the behavior described in the previous paragraph.

In a further elaboration, the weighting of rules 1-6 and also of rule 9 can be made smaller and the weighting of rule 8 can be made larger, if the leak is large or if there is a sudden change in the leak. In this way, to the extent that the respiratory airflow signal is of high quality, ventilatory support will be provided as described in the preceding paragraphs, but to the extent that the respiratory airflow signal is of poor quality and it is difficult to synchronize reliably with the patient's efforts, or to know if the patient's efforts are adequate, ventilatory support will be provided in an orderly manner at a predetermined fixed rate.

As previously mentioned, the Fuzzy Phase Calculation of step 34 also involves fuzzy inference rules relating to respiratory effort. To this end, the invention takes the effort signal $E_{33}(t)$ from the effort sensor 22 and processes additional fuzzy inference rules.

The general method for developing and using these additional fuzzy inference rules for the effort signal is the same as the method described in International Publication Nos. WO 98/12965 and WO 99/61088. Generally, various features, such as the point of start of inspiration, are identified on a graph of effort versus phase, and for each phase, corresponding fuzzy rules are developed. For example, a suitable rule for the point "start of inspiration" could be "effort signal is small and the second derivative of the effort signal with respect to time is large positive." Membership functions, would cause that rule to be maximally activated at or near the start of inspiration. Preferably, the exact phase at the moment of maximal activation should be determined empirically. In the current example, the maximum activation will be at a phase shortly after the actual moment of start of inspiration, say 0.05 revolutions, and this is the best phase to associate with the rule. The more features that are identified and assigned a rule and a phase, the smoother Will be the resultant determination of phase.

The illustrative additional fuzzy inference rules relating to the effort signal $E_{33}(t)$ from the suprasternal effort sensor and the rate of change in the signal, $dE_{33}(t)/dt$, are rules 10-13 provided above.

The fuzzy extents to which the effort signal is in fuzzy sets zero, medium and large are calculated with appropriate membership functions using $E_{33}(t)$. Similarly, the fuzzy extents to which the effort signal is in fuzzy sets increasing moderately, increasing fast, decreasing moderately or decreasing fast are determined with appropriate membership functions using the rate of change of the effort signal, $dE_{33}(t)/dt$. Preferably, the effort signal is normalized for amplitude prior to calculation of degrees of membership, for example, by dividing by the amplitude of the effort signal calculated over a long period compared with a breath, for example, 10-30 seconds.

Continuing with Fig. 2 and the Fuzzy Phase Calculation of step 34, each of the rules in the combined set of fuzzy inference rules is evaluated to determine a degree of activation G(n) by using a standard fuzzy inference method. For example, with respect to rule 12, using one such method assuming a unit weighting of rules, if (a) the degree of truth for the membership function "the effort signal is large" evaluates to 0.6 and (b) the degree of truth for the membership function "the effort signal is decreasing fast" evaluates to 0.4, and a fuzzy logic "AND" operator is applied, then the degree of activation for G(12) would be 0.4.

Additionally, each of the 13 fuzzy inference rules associates a particular rule with a particular phase $\Phi(n)$. For example, as shown above, rule 12 is associated with $\Phi(12) = 0.5$ revolutions. Then, using the degree of activation G(n) for each $\Phi(n)$, a single value representing the instantaneous respiratory phase $\Phi$ is calculated in a defuzzification step using the formula:

$$\Phi = \arctan \left\{ \Sigma \left[ G(n) \sin \Phi(n) \right] / \Sigma \left[ G(n) \cos \Phi(n) \right] \right\}.$$

The phase $\Phi$ is then used in step 36 of Fig. 2 to derive a value from the smooth pressure waveform template $\Pi(\Phi)$.

Step 38, labeled "Amplitude Calculation," involves a calculation of an instantaneous amplitude of pressure support A, chosen to servo-control Instantaneous ventilation to, on average equal a target $V_{TGT}$, by the following formula:

$$A = G \int (V(t) - V_{TGT}) \, dt$$

$$V(t) = 0.5 \, abs \, ( \, f(t) \, )$$

$$A_{MIN} < A < A_{MAX}$$

Finally, in the "Pressure Calculation" step 40, the system calculates the desired degree of ventilatory support P(t) from the calculated amplitude A, a substantial fraction of the patient's airway resistance R, respiratory airflow f(t), and the smooth pressure waveform template $\Pi(\phi)$:

$$P(t) = P_0 + R \, f(t) + A\Pi(\Phi)$$

The Amplitude Calculation and Pressure Calculation steps as just described are comparable to the steps in International Publication Nos. WO 98/12965 and WO 99/61088.

In the preferred embodiment of the invention, the effort sensor is an accessory that can be added or removed at will from a ventilator such as an AutoVPAP system in order to achieve improved synchronization with the patient, If the effort sensor falls off the patient, fails, is electrically unplugged or is otherwise removed, then rules (10) to (13) will have no effect on the phase determination or, in other words, the rules will have no degree of activation. In this event, the device will behave as a simple servo-controlled ventilator such as the AutoVPAP device. The patient will continue to be ventilated, with ventilatory support provided approximately in phase with respiratory airflow and therefore approximately in phase with respiratory effort. Thus, the degree of support will be sufficient to guarantee that instantaneous ventilation on average equals or exceeds the target ventilation, but the precise timing information and improved immunity to the effects of leaks will be lost.

While the described embodiment of the present invention makes use of a suprasternal effort sensor, effort signals from other sensors may also be utilized. Other forms of effort signal include, for example, an effort signal derived from esophageal pressure generated by a pressure transducer implanted in the chest that sends a signal via telemetry. Alternatively, an electromyogram signal from an electromyograph could be used without requiring any modification to the invention. Moreover, multiple effort sensors can be utilized simply with the addition of extra fuzzy inference rules relating to the additional effort sensors.

Although the invention has been described with reference to particular embodiments, it is to be understood that these embodiments are merely illustrative of an application of the principles of the invention. For example, one embodiment of the invention might utilize a set of rules to determine phase in which some of the rules determine phase based solely upon the magnitude of the respiratory airflow and the rate of change of an effort signal. Alternatively, some rules may determine phase based upon the magnitude of the effort signal and the rate of change of respiratory airflow. Numerous modifications, in addition to the illustrative embodiments of the invention discussed herein may be made and other arrangements may be devised without departing from the scope of the invention as defined by the accompanying claims.

**Claims**

1.  An apparatus for providing synchronized ventilatory support to a patient comprising:

    at least one sensor (14, 15) to generate a respiratory airflow signal (f(t));
    at least one sensor (22) to generate a respiratory effort signal ($E_{ss}(t)$), wherein the effort sensor (22) is not dependent on measured airflow;
    a processor (16) for analyzing said respiratory airflow and said effort signals for determining the instantaneous respiratory phase ($\Phi$) of the patient and for generating a desired pressure request signal (P(t)) as a function of said instantaneous respiratory phase ($\Phi$) and a desired ventilation; and
    a servo-controlled blower (10) to provide pressurized air to said patient in accordance with said pressure request signal (P(t)).

2. The apparatus of claim 1 wherein said respiratory effort sensor is selected from a group of effort sensors comprising:

    (a) a suprasternal notch sensor;
    (b) an esophageal pressure effort sensor; and
    (c) an electromyograph.

3. The apparatus of claim 1 or claim 2 wherein said processor (16) evaluates fuzzy inference rules relating to said respiratory effort signal ($E_{ss}$(t)).

4. The apparatus of claim 3 wherein said processor (16) evaluates fuzzy inference rules relating to the rate of change ($dE_{ss}$(t)/dt) of said respiratory effort signal ($E_{ss}$(t)).

5. The apparatus of claim 1 or 4 wherein said processor (16) evaluates fuzzy logic inference rules relating to the respiratory airflow (f(t)).

6. The apparatus of claim 5 wherein said processor (16) evaluates fuzzy logic inference rules relating to the rate of change (df(t)/dt) of the respiratory airflow (f(t)).

7. The apparatus of claim 4 wherein said fuzzy inference rules include:-

    (i) at least one rule selected from a group of rules including:

        (a) If the effort signal ($E_{ss}$(t)) is zero and increasing fast, then the phase ($\Phi$) is about 0 revolutions;
        (b) If the effort signal ($E_{ss}$(t)) is medium and increasing moderately, then the phase ($\Phi$) is about 0.2 revolutions;
        (c) If the effort signal ($E_{ss}$(t)) is large and decreasing fast, then the phase ($\Phi$) is about 0.5 revolutions; and
        (d) If the effort signal ($E_{ss}$(t)) is medium and decreasing moderately, then the phase ($\Phi$) is about 0.7 revolutions, or

    (ii) a rule in which the start of inspiration is associated with approximately zero respiratory effort which is increasing fast; or
    (iii) a rule in which mid-inspiration is associated with medium respiratory effort which is increasing moderately; or
    (iv) a rule in which the beginning of expiration is associated with large respiratory effort which is decreasing fast; or
    (v) a rule in which mid-expiration is associated with medium respiratory effort which is decreasing moderately.

8. The apparatus of claims 1 and 3 to 6 wherein said fuzzy inference rules include at least one rule selected form a group of rules comprising:

    (a) If the airflow (f(t)) is zero and increasing fast, then the phase ($\Phi$) is about 0 revolutions;
    (b) If the airflow (f(t)) is large positive and steady, then the phase ($\Phi$) is about 0.25 revolutions;
    (c) If the airflow (f(t)) is zero and falling fast, then the phase ($\Phi$) is about 0.5 revolutions;
    (d) If the airflow (f(t)) is large negative and steady, then the phase ($\Phi$) is about 0.75 revolutions;
    (e) If the airflow (f(t)) is zero and steady and the 5-second low-pass filtered absolute value of the respiratory airflow is large, then the phase ($\Phi$) is about 0.9 revolutions;
    (f) If the airflow (f(t)) is positive and the phase is expiratory, then the phase ($\Phi$) is about 0.1 revolutions;
    (g) If the airflow (f(t)) is negative and the phase is inspiratory, then the phase ($\Phi$) is about 0.6 revolutions;
    (h) If the 5-second low-pass filtered absolute value of the respiratory airflow (f(t)) is small, then the phase ($\Phi$) in the respiratory cycle is increasing at a fixed rate equal to the patient's expected respiratory rate; and
    (i) If the 5-second low-pass filtered absolute value of the respiratory airflow (f(t)) is large, then the phase ($\Phi$) In the respiratory cycle is increasing at a steady rate equal to the existing rate of change of phase, low-pass filtered with a time constant of 20 seconds.

9. The apparatus of any of claims 1 to 8 wherein the desired ventilation has a non-zero minimum value and a maximum value.

10. The apparatus of claim 9 wherein the generation of said desired pressure request signal (P(t)) includes deriving an error value that is a function of the difference between calculated patient ventilation and a target value.

**Patentansprüche**

1. Vorrichtung zum Bereitstellen von synchronisierter Beatmungshilfe für einen Patienten, umfassend:

   zumindest einen Sensor (14, 15) zum Generieren eines Atemluftstromsignals (f(t));
   zumindest einen Sensor (22) zum Generieren eines Atemanstrengungssignals ($E_{ss}$(t)), wobei der Anstrengungssensor (22) nicht vom gemessenen Luftstrom abhängig ist;
   einen Prozessor (16) zum Analysieren des Atemluftstromsignals und des Atemanstrengungssignals zum Bestimmen der momentanen Atemphase ($\phi$) des Patienten und zum Generieren eines Anforderungssignals (P(t)) für den gewünschten Druck als eine Funktion der momentanen Atemphase ($\phi$) und einer gewünschten Beatmung; und
   ein servogesteuertes bzw. -geregeltes Gebläse (10), um dem Patienten Druckluft gemäß dem Druckanforderungssignal (P(t)) bereitzustellen.

2. Vorrichtung nach Anspruch 1, wobei der Atemanstrengungssensor aus einer Gruppe von Anstrengungssensoren ausgewählt ist, die umfasst:

   (a) einen Jugulum-Sensor;
   (b) einen Speiseröhrendruck-Anstrengungssensor; und
   (c) einen Elektromyograph.

3. Vorrichtung nach Anspruch 1 oder Anspruch 2, wobei der Prozessor (16) Fuzzy-Inferenzregeln auswertet, die das Atemanstrengungssignal ($E_{ss}$(t)) betreffen.

4. Vorrichtung nach Anspruch 3, wobei der Prozessor (16) Fuzzy-Inferenzregeln auswertet, welche die Veränderungsrate ($dE_{ss}$(t)/dt) des Atemanstrengungssignals ($E_{ss}$(t)) betreffen.

5. Vorrichtung nach Anspruch 1 oder 4, wobei der Prozessor (16) Fuzzy-Logik-Inferenzregeln auswertet, die den Atemluftstrom (f(t)) betreffen.

6. Vorrichtung nach Anspruch 5, wobei der Prozessor (16) Fuzzy-Logik-Interferenzregeln auswertet, welche die Veränderungsrate (df(t)/dt) des Atemluftstroms (f(t)) betreffen.

7. Vorrichtung nach Anspruch 4, wobei die Fuzzy-Logik-Interferenzregeln enthalten:

   (i) zumindest eine Regel, die aus einer Gruppe von Regeln ausgewählt ist, die enthalten:

   (a) Wenn das Anstrengungssignals ($E_{ss}$(t)) Null ist und schnell ansteigt, dann beträgt die Phase ($\phi$) ca. 0 Umdrehungen;
   (b) Wenn das Anstrengungssignals ($E_{ss}$(t)) mittel ist und moderat ansteigt, dann beträgt die Phase ($\phi$) ca. 0,2 Umdrehungen;
   (c) Wenn das Anstrengungssignals ($E_{ss}$(t)) groß ist und schnell abnimmt, dann beträgt die Phase ($\phi$) ca. 0,5 Umdrehungen;
   (d) Wenn das Anstrengungssignals ($E_{ss}$(t)) mittel ist und moderat abnimmt, dann beträgt die Phase ($\phi$) ca. 0,7 Umdrehungen; oder

   (ii) eine Regel, bei welcher der Beginn der Einatmung mit näherungsweise null Atemanstrengung assoziiert wird, die schnell ansteigt; oder
   (iii) eine Regel, bei der eine Zwischeneinatmung mit mittlerer Atemanstrengung assoziiert wird, die moderat ansteigt; oder
   (iv) eine Regel, bei welcher der Anfang der Ausatmung mit großer Atemanstrengung assoziiert wird, die schnell abnimmt; oder
   (v) eine Regel, bei der eine Zwischenausatmung mit mittlerer Atemanstrengung assoziiert wird, die moderat abnimmt.

8. Vorrichtung nach Anspruch 1 und 3 bis 6, wobei die Fuzzy-Logik-Interferenzregeln zumindest eine Regel enthalten, die aus einer Gruppe von Regeln ausgewählt ist, die enthalten:

(a) Wenn der Luftstrom (f(t)) Null ist und schnell ansteigt, dann beträgt die Phase ($\phi$) ca. 0 Umdrehungen;
(b) Wenn der Luftstrom (f(t)) stark positiv und stetig ist, dann beträgt die Phase ($\phi$) ca. 0,25 Umdrehungen;
(c) Wenn der Luftstrom (f(t)) Null ist und schnell abnimmt, dann beträgt die Phase ($\phi$) ca. 0,5 Umdrehungen;
(d) Wenn der Luftstrom (f(t)) stark negativ und stetig ist, dann beträgt die Phase ($\phi$) ca. 0,75 Umdrehungen;
(e) Wenn der Luftstrom (f(t)) Null und stetig ist und der 5-sekündige tiefpassgefilterte Absolutwert des Atemluftstroms groß ist , dann beträgt die Phase ($\phi$) ca. 0,9 Umdrehungen;
(f) Wenn der Luftstrom (f(t)) positiv und die Phase Ausatmen ist, dann beträgt die Phase ($\phi$) ca. 0,1 Umdrehungen;
(g) Wenn der Luftstrom (f(t)) negativ und die Phase Einatmen ist, dann beträgt die Phase ($\phi$) ca. 0,6 Umdrehungen;
(h) Wenn der 5-sekündige tiefpassgefilterte Absolutwert des Atemluftstroms (f(t)) klein ist, dann nimmt die Phase ($\phi$) in dem Atemzyklus mit einer festen Rate zu, die gleich der erwarteten Atemrate des Patienten ist; und
(i) Wenn der 5-sekündige tiefpassgefilterte Absolutwert des Atemluftstroms (f(t)) groß ist, dann nimmt die Phase ($\phi$) in dem Atemzyklus mit einer stetigen Rate zu, die gleich der bestehenden Veränderungsrate der Phase ist, und zwar tiefpassgefiltert mit einer Zeitkonstante von 20 Sekunden.

9. Vorrichtung nach einem der Ansprüche 1 bis 8, wobei die gewünschte Beatmung einen Minimumwert von nicht Null und einen Maximumwert aufweist.

10. Vorrichtung nach Anspruch 9, wobei das Generieren des gewünschten Druckanforderungssignals (P(t)) das Ableiten eines Fehlerwerts beinhaltet, der eine Funktion der Differenz zwischen der berechneten Beatmung des Patienten und einem Zielwert ist.

**Revendications**

1. Appareil destiné à fournir une assistance ventilatoire synchronisée à un patient, comprenant :

au moins un capteur (14, 15) pour générer un signal de flux d'air respiratoire (f(t)) ;
au moins un capteur (22) pour générer un signal d'effort respiratoire ($E_{ss}$(t)), le capteur d'effort (22) n'étant pas dépendant du flux d'air mesuré ;
un processeur (16) pour analyser ledit flux d'air respiratoire et lesdits signaux d'effort pour déterminer la phase respiratoire instantanée ($\Phi$) du patient et pour générer un signal de demande de pression désirée (P(t)) en fonction de ladite phase respiratoire instantanée ($\Phi$) et d'une ventilation désirée ; et
une soufflante asservie (10) pour fournir de l'air pressurisé audit patient en fonction dudit signal de demande de pression (P(t)).

2. Appareil selon la revendication 1, dans lequel ledit capteur d'effort respiratoire est sélectionné dans un groupe de capteurs d'effort comprenant :

(a) un capteur de fourchette sternale;
(b) un capteur de pression oesophagienne ; et
(c) un électromyographe.

3. Appareil selon la revendication 1 ou 2, dans lequel ledit processeur (16) évalue des règles d'inférence floue relatives audit signal d'effort respiratoire ($E_{ss}$(t)).

4. Appareil selon la revendication 3, dans lequel ledit processeur (16) évalue des règles d'inférence floue relatives à la vitesse de changement ($dE_{ss}$(t)/dt), dudit signal d'effort respiratoire ($E_{ss}$(t)).

5. Appareil selon la revendication 1 ou 4, dans lequel ledit processeur (16) évalue des règles d'inférence floue relatives au flux d'air respiratoire (f(t)).

6. Appareil selon la revendication 5, dans lequel ledit processeur (16) évalue des règles d'inférence floue relatives à la vitesse de changement (df(t)/dt) du flux d'air respiratoire (f(t)).

**7.** Appareil selon la revendication 4, dans lequel lesdites règles d'inférence floue comprennent :

(i) au moins une règle choisie dans le groupe de règles comprenant :

(a) Si le signal d'effort ($E_{ss}(t)$) est égal à zéro et augmente rapidement, la phase (Φ) est alors d'environ 0 révolutions ;
(b) Si le signal d'effort ($E_{ss}(t)$) est moyen et augmente modérément, la phase (Φ) est alors d'environ 0,2 révolutions ;
(c) Si le signal d'effort ($E_{ss}(t)$) est grand et diminue rapidement, la phase (Φ) est alors d'environ 0,5 révolutions ; et
(d) Si le signal d'effort ($E_{ss}(t)$) est moyen et diminue modérément, la phase (Φ) est alors d'environ 0,7 révolutions, ou bien

(ii) une règle dans laquelle le début de l'inspiration est associé à un effort respiratoire de sensiblement zéro qui augmente rapidement ; ou
(iii) une règle dans laquelle le milieu de l'aspiration est associé à un effort respiratoire moyen qui augmente modérément ; ou
(iv) une règle dans laquelle le début de l'expiration est associé à un grand effort respiratoire qui diminue rapidement ; ou
(v) une règle dans laquelle le milieu de l'expiration est associé à un effort respiratoire moyen qui diminue modérément.

**8.** Appareil selon les revendications 1 et 3 à 6, dans lequel lesdites règles d'inférence floue comprennent au moins une règle choisie dans un groupe de règles comprenant :

(a) Si le flux d'air (f(t)) est égal à zéro et augmente rapidement, la phase (Φ) est alors d'environ 0 révolutions ;
(b) Si le flux d'air (f(t)) est positif grand et constant, la phase (Φ) est alors d'environ 0,25 révolutions ;
(c) Si le flux d'air (f(t)) est égal à zéro et tombe rapidement, la phase (Φ) est alors d'environ 0,5 révolutions ;
(d) Si le flux d'air (f(t)) est négatif grand et constant, la phase (Φ) est alors d'environ 0,75 révolutions ;
(e) Si le flux d'air (f(t)) est égal à zéro et constant et si la valeur absolue de 5 secondes, filtrée par un filtre passe-bas, du flux d'air respiratoire est grande, la phase (Φ) est alors d'environ 0,9 révolutions ;
(f) Si le flux d'air (f(t)) est positif et que la phase est expiratoire, la phase (Φ) est alors d'environ 0,1 révolutions ;
(g) Si le flux d'air (f(t)) est négatif et que la phase est inspiratoire, la phase (Φ) est alors d'environ 0,6 révolutions ;
(h) Si la valeur absolue du flux d'air respiratoire (f(t)) filtrée par un filtre passe-bas de 5 secondes est petite, alors la phase (Φ) dans le cycle respiratoire augmente à une vitesse fixe égale à la vitesse respiratoire attendue du patient ; et
(i) Si la valeur absolue du flux d'air respiratoire (f(t)) filtrée par un filtre passe-bas de 5 secondes est grande, alors la phase (Φ) dans le cycle respiratoire augmente à une vitesse constante égale à la vitesse existante du changement de phase, filtrée par un filtre passe-bas avec une constante de temps de 20 secondes.

**9.** Appareil selon l'une quelconque des revendications 1 à 8, dans lequel la ventilation désirée a une valeur minimum différente de zéro et une valeur maximum.

**10.** Appareil selon la revendication 9, dans lequel la génération dudit signal de demande de pression désirée (P(t)) inclut la dérivation d'une valeur d'erreur qui est fonction de la différence entre la ventilation calculée du patient et une valeur cible.

Fig. 1

Fig. 2

EP 1 810 708 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9812965 A
- EP 0452001 A
- US 5803065 A
- EP 1083953 A
- US 6532959 B
- WO 9961088 A
- US 6758216 B
- US 6445942 B